(19) Europäisches Patentamt
European Patent Office
Office européen des brevets

(11) Veröffentlichungsnummer: **0 017 617**
**A2**

# (12) EUROPÄISCHE PATENTANMELDUNG

(21) Anmeldenummer: 80810093.7

(22) Anmeldetag: 21.03.80

(51) Int. Cl.³: C 07 D 498/10
C 08 K 5/35
//(C07D498/10, 263/00, 221/00)

(30) Priorität: 27.03.79 CH 2826/79

(43) Veröffentlichungstag der Anmeldung:
15.10.80 Patentblatt 80/21

(84) Benannte Vertragsstaaten:
CH DE FR GB IT

(71) Anmelder: CIBA-GEIGY AG
Patentabteilung Postfach
CH-4002 Basel(CH)

(72) Erfinder: Slongo, Mario, Dr.
Unterwartweg 27
CH-4132 Muttenz(CH)

(72) Erfinder: Nikles, Erwin, Dr.
Bodenackerstrasse 14
CH-4410 Liestal(CH)

## (54) Polyalkylpiperidin-spirooxazoine, ihre Verwendung als Lichtschutzmittel und mit diesen Verbindungen stabilisierter Kunststoff.

(57) Verbindungen der Formel

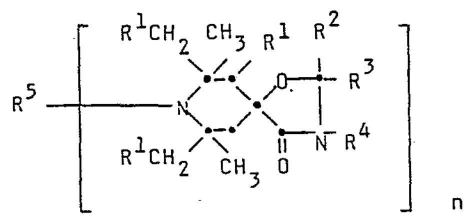

I

und deren Säureadditions-Salze, worin n eine ganze Zahl von 1 bis 4 ist, $R_1$ Wasserstoff oder $CH_3$ ist, $R_2$ und $R_3$ unabhängig voneinander Wasserstoff, $C_1$-$C_{18}$-Alkyl, unsubstituiertes oder durch Chlor oder $C_1$-$C_4$-Alkyl substituiertes $C_6$-$C_{10}$-Aryl oder $C_7$-$C_9$-Aralkyl bedeuten oder $R^2$ und $R^3$ zusammen mit dem C-Atom, an das sie gebunden sind, einen Cycloalkan- oder Alkylcycloalkanrest mit 5-20 C-Atomen oder einen Polyalkylpiperidinrest bilden, $R^4$ einen in Anspruch 1 definierten einwertigen Rest und $R^5$ einen in Anspruch 1 definierten ein- bis vierwertigen Rest darestellt, sind wirkungsvolle Lichtschutzmittel für Kunststoffe. Ihre Herstellung geschieht durch N-Substitution der Verbindungen, in denen $R^4$ und $R^5$ Wasserstoff ist.

EP 0 017 617 A2

CIBA-GEIGY AG
Basel (Schweiz) 3-12278/S/+

BEZEICHNUNG GEÄNDERT
siehe Titelseite

Polyalkylpiperidin-spirooxazolone und ihre Verwendung als Lichtschutzmittel

Es ist bekannt, dass in 4-Stellung substituierte Polyalkylpiperidine wertvolle Lichtschutzmittel für organische Materialien, vor allem für Kunststoffe sind. So wurden in der DE-OS 2 606 026 auch schon Polyalkylpiperidin-4-spirooxazolone als Lichtschutzmittel beschrieben, deren Piperidin-Stickstoffatom durch Wasserstoff, Sauerstoff, Hydroxyl oder Niederalkyl substituiert ist.

Diese bekannten Polyalkylpiperidinderivate sind ausgezeichnete Lichtschutzmittel für Kunststoffe. Für bestimmte Verwendungszwecke ist jedoch die Flüchtigkeit und Migrationstendenz dieser bekannten Verbindungen zu hoch. Dies ist vor allem der Fall, wenn der Kunststoff in dünnen Schichten verwendet wird, wie z.B. in Fasern, Folien oder Ueberzügen. Viele der bekannten Spiroverbindungen sind ausserdem in Polymeren schlecht löslich, was zu einer ungenügenden Verträglichkeit in gewissen Kunststoffen führt. Es wurde nun gefunden, dass bestimmte Polyalkylpiperidin-4-spirooxazolone eine wesentlich geringere Flüchtigkeit und Migrationstendenz besitzen als die bekannten Polyalkylpiperidin-4-spirooxazolone und in gewissen Kunststoffen auch eine bessere Verträglichkeit aufweisen. Es handelt sich dabei um die Verbindungen der Formel I

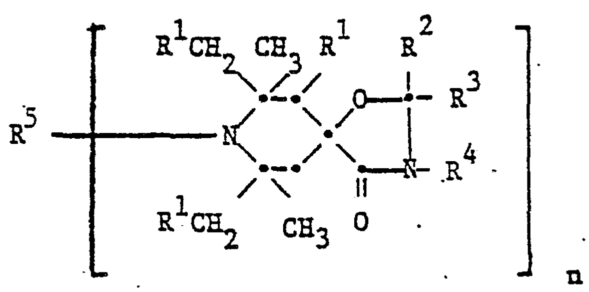

I

und deren Säureadditionssalze, worin n eine ganze Zahl von 1 bis 4 ist, $R^1$ Wasserstoff oder $CH_3$ ist, $R^2$ und $R^3$ unabhängig voneinander Wasserstoff, $C_1$-$C_{18}$-Alkyl, unsubstituiertes oder durch Chlor oder $C_1$-$C_4$-Alkyl substituiertes $C_6$-$C_{10}$-Aryl oder $C_7$-$C_9$-Aralkyl bedeuten oder $R^2$ und $R^3$ zusammen mit dem C-Atom, an das sie gebunden sind, einen Cycloalkan- oder Alkylcycloalkanrest mit 5-20 C-Atomen oder einen Polyalkylpiperidinrest bilden, $R^4$ Wasserstoff, $C_1$-$C_8$-Alkyl, Allyl, Propargyl, Glycidyl oder unsubstituiertes oder durch $C_1$-$C_4$-Alkyl substituiertes $C_7$-$C_9$-Aralkyl ist, $R^5$ wenn n=1 ist, Wasserstoff, $C_1$-$C_8$-Alkyl, $C_3$-$C_8$-Alkenyl, $C_3$-$C_5$-Alkinyl, unsubstituiertes oder durch $C_1$-$C_4$-Alkyl substituiertes $C_7$-$C_9$-Aralkyl oder einen Rest $-CH_2-CH(R^6)-OR$ darstellt, worin $R^6$ H, $CH_3$, $C_2H_5$ oder Phenyl ist und $R^7$ Wasserstoff oder den Acylrest einer aliphatischen, cycloaliphatischen oder aromatischen Monocarbonsäure mit bis zu 18 C-Atomen bedeutet, und wenn n=2 ist, 1,4-Buten-2-ylen, m- oder p-Xylylen oder einen Rest der Formel $-CH_2-CH(R^6)-O-R^8-O-CH(R^6)-CH_2-$ darstellt, worin $R^6$ die oben angegebene Bedeutung hat und $R^8$ den Diacylrest einer aliphatischen, cycloaliphatischen oder aromatischen Dicarbonsäure mit 3-14 C-Atomen bedeutet, und wenn n=3 ist, einen Rest der Formel

$$\begin{array}{l} -CH_2-CH(R^6)-O \diagdown \\ \qquad\qquad\qquad\qquad\quad R^9-O-CH(R^6)-CH_2- \\ -CH_2-CH(R^6)-O \diagup \end{array}$$

darstellt, worin $R^6$ die oben gegebene Bedeutung hat und $R^9$ den Triacylrest einer aliphatischen oder aromatischen Tricarbonsäure mit 4-18 C-Atomen bedeutet, und wenn n=4 ist, einen Rest der Formel

$$\begin{array}{ccc} -CH_2-CH(R^6)-O & & O-CH(R^6)-CH_2- \\ & \diagdown \; R^{10} \; \diagup & \\ & \diagup \qquad \diagdown & \\ -CH_2-CH(R^6)-O & & O-CH(R^6)-CH_2- \end{array}$$

darstellt, worin $R^6$ die oben gegebene Bedeutung hat und $R^{10}$ den

Tetracylrest einer aliphatischen oder aromatischen Tetracarbonsäure mit 6-20 C-Atomen bedeutet, wobei im Fall, dass $R^5$ Wasserstoff oder $C_1$-$C_4$-Alkyl ist, $R^4$ nicht Wasserstoff sein kann.

$R^2$ und $R^3$ als Alkyl können unverzweigtes oder verzweigtes Alkyl sein, beispielsweise Methyl, Aethyl, Propyl, Isobutyl, Hexyl, 2-Aethylhexyl, Isooctyl, Decyl, Dodecyl oder Octadecyl. $R^2$ und $R^3$ als unsubstituiertes oder substituiertes Aryl können beispielsweise Phenyl, Naphthyl, Chlorphenyl, Tolyl oder 4-Butylphenyl sein. $R^2$ und $R^3$ als Aralkyl können beispielsweise Benzyl, Phenyläthyl oder Phenylpropyl sein.

Bilden $R^2$ und $R^3$ zusammen mit dem C-Atom, an das sie gebunden sind, einen Cycloalkan- oder Alkylcycloalkanrest, so kann dies z.B. ein Cyclopentan-. Cyclohexan-, Methylcyclohexan-, Cyclodecan- Cyclooctan-, Cyclododecan- oder Dimethylcyclododecanrest sein.

Bevorzugt sind $R^2$ und $R^3$ unabhängig voneinander Alkyl mit 1-12 C-Atomen oder Phenyl oder $R^2$ und $R^3$ bilden zusammen mit dem C-Atom, an das sie gebunden sind, einen $C_6$-$C_{12}$-Cycloalkanrest.

$R^4$ und $R^5$ als Alkyl können z.B. Methyl, Aethyl, Propyl, Butyl, Hexyl, 2-Aethylhexyl oder n-Octyl sein.

$R^5$ als Alkenyl kann z.B. Allyl, Methallyl, Butenyl, Hexenyl oder Octenyl sein. $R^4$ und $R^5$ als Aralkyl können z.B. Benzyl, Phenyläthyl oder 4-Methylbenzyl sein.

$R^7$ als Carbonsäurerest kann z.B. der Rest von Essigsäure, Acrylsäure, Propionsäure, Buttersäure, Caprylsäure, Palmitinsäure, Oelsäure, Stearinsäure, Benzoesäure, Phenylessigsäure oder Naphthoesäure sein. $R^8$ als Dicarbonsäurerest kann z.B. der zweiertige Rest von Malon-, Bernstein-, Malein-, Adipin-, Itacon-, Sebacin-, Kork-,

Phthal-, Terephthal- ,Isophthal-, 4,4'-Diphenyl-, Bicyclo[2.2.1]hepten- oder Bicyclo[2.2.1]heptan-2,3-dicarbonsäure sein.

$R^9$ als Tricarbonsäurerest kann z.B. der dreiwertige Rest von Tricarballylsäure, Nitrilotriessigsäure, Aconitsäure oder Trimellitsäure sein. $R^{10}$ als Tetracarbonsäurerest kann z.B. der vierwertige Rest von Methylendimalonsäure, Methylendiphthalsäure oder Pyromellitsäure sein.

Bevorzugt sind Verbindungen der Formel I, worin $R^1$ Wasserstoff ist sowie Verbindungen der Formel I, worin n 1 oder 2 ist.

Bevorzugt sind weiterhin Verbindungen der Formel I, worin n=1 ist, $R^4$ Wasserstoff ist und $R^5$ Allyl, Benzyl, 2-Hydroxyäthyl oder 2-Hydroxypropyl bedeutet, sowie Verbindungen der Formel I, worin n=1 ist, $R^4$ Allyl oder Benzyl bedeutet und $R^5$ Wasserstoff ist.

Die vorliegende Erfindung umfasst auch die Salze von Verbindungen der Formel I, die durch Addition von Säuren in maximal den Piperidingruppen äquivalenten Mengen entstehen. Solche Säuren können anorganische Säuren sein, wie z.B. Schwefel-, Salz- oder Phosphorsäure, organische Carbonsäure wie Ameisen-, Essig-, Oxal-, Malein-, Benzoe- oder Salicylsäure, organische Sulfonsäuren wie Methan- oder p-Toluolsulfonsäure oder organische phosphorhaltige Säuren wie Diphenylphosphorsäure, Methanphosphonsäure oder Diphenylphosphinsäure.

Beispiele für Verbindungen der Formel I sind:

8-Allyl-7,7,9,9-tetramethyl-2,2-dimethyl-1-oxa-3,8-diaza-4-oxo-spiro-[4,5]decan,
8-Benzyl-7,7,9,9-tetramethyl-2,2-dimethyl-1-oxa-3,8-diaza-4-oxo-spiro-[4,4]decan,

8-(2-Hydroxyäthyl)-7,7,9,9-tetramethyl-2-methyl-2-äthyl-1-oxa-3,8-diaza-4-oxo-spiro[4,5]decan,

8-(2-Acetoxyäthyl)-7,7,9,9-tetramethyl-2-methyl-2-äthyl-1-oxa-3,8-diaza-4-oxo-spiro[4,5]decan,

8-(2-Benzoyloxyäthyl)-7,7,9,9-tetramethyl-2-methyl-2-phenyl-1-oxa-3,8-diaza-4-oxo-spiro[4,5]decan,

8-(2-Phenyläthyl)-7,7,9,9,-tetramethyl-2-phenyl-1-oxa-3,8-diaza-4-oxospiro[4,5]decan,

8-(2-Phenyl-2-hydroxyäthyl)-7,7,9,9-tetramethyl-2,2-dibutyl-1-oxa-3,8-diaza-4-oxo-spiro[4,5]decan,

8-(2-Stearoyloxyäthyl)-6,7,9-trimethyl-7,9-diäthyl-2-methyl-2-benzyl-1-oxa-3,8-diaza-4-oxo-spiro[4,5]decan,

1,4-Bis(7,7,9,9-tetramethyl-2,2-diäthyl-1-oxa-3,8-diaza-4-oxo-spiro-[4,5]decyl)buten-2,

α,α'-Bis(7,7,9,9-tetramethyl-2-propyl -1-oxa-3,8-diaza-4-oxo-spiro-[4,5]decyl-8)-m-xylylen,

Di-[2-(7,7,9,9-tetramethyl-2,2-dibutyl-1-oxa-3,8-diaza-4-oxo-spiro-[4,5]decyl-8)-äthyl]-succinat,

Di-[2-(7,7,9,9-tetramethyl-2,2-dibutyl-1-oxa-3,8-diaza-4-oxo-spiro-[4,5]decyl-8)-propyl]-adipat,

Di-[2-(7,7,9,9-tetramethyl-2,2-dibutyl-1-oxa-3,8-diaza-4-oxo-spiro-[4,5]decyl-8)-äthyl]-isophthalat,

Tri-[2-(7,7,9,9-tetra-ethyl-2,2-dibutyl-1-oxa-3,8-diaza-4-oxo-spiro-[4,5]decyl-8)-äthyl]-trimellitat,

Tetra-[2-(7,7,9,9-tetramethyl-2,2-dibutyl-1-oxa-3,8-diaza-4-oxo-spiro[4,5]decyl-8)-äthyl]pyromellitat,

3-Allyl-2,2,4,4-tetramethyl-7-oxa-3,14-diaza-15-oxo-dispiro[5,1,5,2]-pentadecan,

Di-[2-(2,2,4,4-tetramethyl-4-oxa-3,14-diaza-15-oxo-dispiro[5,1,5,2]-pentadecyl-3)-äthyl]-sebacat,

3-Benzyl-2,2,4,4-tetramethyl-7-oxa-3,20-diaza-21-oxo-dispiro[5,1,11,2]-heneicosan,

1,4-Di-(2,2,4,4-tetramethyl-7-oxa-3,20-diaza-21-oxo-dispiro[5,1,11,2]-heneicosyl-3)-buten-2,

2,2,4,4-Tetramethyl-7-oxa-3-aza-20-(methyl)aza-21-oxo-dispiro-[5.1,11,2]-heneicosan,

2,2,4,4-Tetramethyl-7-oxa-3-aza-20-(benzyl)-aza-21-oxo-dispiro-[5,1,11,2]-heneicosan,

2,2,4,4-Tetramethyl-7-oxa-3-aza-20-(2-propenyl)aza-21-oxo-dispiro-[5,1,11,2]-heneicosan,

2,2,4,4-Tetramethyl-7-oxa-3-aza-20-(butyl)aza-21-oxo-dispiro-[5,1,11,2]-heneicosan,

2,2,4,4-Tetramethyl-7-oxa-3-aza-20-(glycidyl)aza-21-oxo-dispiro-[5,1,11,2)-heneicosan.

Die Verbindungen der Formel I, worin $R^4$ und $R^5$ Wasserstoff sind, sind aus der DE-OS 2 606 026 bekannt. Aus diesen Verbindungen erhält man die erfindungsgemässen Verbindungen der Formel I durch Einführung des Substituenten $R^4$ am Oxazolon-Stickstoff oder durch Einführung des Substituenten $R^5$ am Piperidin-Stickstoff oder durch stufenweise Einführung beider Substituenten.

Die Einführung von $R^4$ geschieht vorzugsweise dadurch, dass man die NH-Verbindung durch Reaktion mit starken Alkalibasen in die entsprechenden Alkaliverbindungen überführt, welche anschliessend mit Alkyl-, Allyl-, Propargyl-, Glycidyl- oder Aralkylhalogeniden umgesetzt werden. Die Ueberführung in die Alkaliverbindungen geschieht vorzugsweise im sogenannten Phasentransferverfahren, wobei man ein Lösungsmittel verwendet, in welchem die Alkalibasen nicht löslich sind, so dass diese als feste Phase vorliegen. Beispiele für verwendbare Alkalibasen sind Alkali-oxide, -amide, -hydride, -alkoxide, vorzugsweise jedoch Alkalihydroxyde wie Kaliumhydroxyd. Beispiele für verwendbare Lösungsmittel sind Diäthyläther, Tetrahydrofuran, Dioxan, Benzol oder Toluol. Bei der anschliessenden Umsetzung mit Halogenverbindungen entsteht Alkalihalogenid, das in diesen Lösungsmitteln ebenfalls unlöslich ist und daher durch Filtration einfach entfernt werden kann. Beispiele für verwendbare Halogenverbindungen sind Methyliodid, Butylbromid, Allylchlorid, Benzylchlorid oder Xylylendibromid.

Die Einführung von $R^5$ geschieht durch direkte Umsetzung der NH-Verbindung mit den Substitutionsreagentien. Als Substitutionsreagentien sind sind wiederum die Halogenide geeignet, wie Alkyl-, Alkenyl-, Alkinyl- oder Aralkylhalogenide. Hierbei kann man dem Reaktionsgemisch schwache Basen zusetzen, wie z.B. Alkalicarbonate oder Erdalkalioxide, die den entstehenden Halogenwasserstoff binden. Verbindungen der Formel I, worin $R^5$ ein Hydroxyalkyl- bzw. Hydroxyaralkylrest ist, können durch Umsetzung der NH-Verbindungen mit den entsprechenden Alkylenoxiden oder mit Styroloxid hergestellt werden. Diese Hydroxylverbindungen können mit Mono-, Di-, Tri- oder Tetracarbonsäuren verestert werden, wodurch man die entsprechenden N-Acyloxyalkyl- bzw. -aralkylverbindungen erhält.

Will man sowohl an den Oxazolon-Stickstoff wie an den Piperidin-Stickstoff Substituenten einführen, so führt man vorteilhafterweise zuerst $R^4$ und anschliessend $R^5$ nach den oben genannten Methoden ein.

Die Verbindungen der Formel I sind als Stabilisatoren für Kunststoffe verwendbar, sie zeichnen sich vor allem durch eine hervorragende Lichtschutzwirkung aus. Beispiele für solche Kunststoffe sind die in der DT-OS 2 456 864 auf den Seiten 12-14 aufgeführten Polymeren.

Von besonderer Bedeutung ist die Stabilisierung von Polyolefinen, Styrolpolymerisaten und von Polyurethanen, für die sich die Verbindungen der Formel I hervorragend eignen. Beispiele hierfür sind Polyäthylen hoher und niedriger Dichte, Polypropylen, Aethylen--Propylen-Copolymerisate, Polystyrol, Styrol-Butadien-Acrylnitril-Copolymerisate, Mischungen von Polyolefinen oder von Styrolpolymerisaten, Polyurethane auf Polyäther- oder Polyesterbasis in Form von Lacken, Elastomeren oder Schaumstoffen.

Die Stabilisatoren werden den Kunststoffen in einer Konzentration von 0,01 bis 5 Gew.%, berechnet auf das zu stabilisierende Material, zugesetzt. Vorzugsweise werden 0,1 bis 1 Gew.% der Verbindungen, berechnet auf das zu stabilisierende Material, in dieses eingearbeitet.

Die Einarbeitung kann beispielsweise durch Einmischen mindestens eines der erfindungsgemässen Lichtschutzmittel und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen.

Ausser den Verbindungen der Formel I können den Kunststoffen auch noch andere, bekannte Stabilisatoren zugesetzt werden. Dies können z.B. Antioxydantien, Lichtschutzmittel oder Metalldesaktivatoren sein, oder auch Costabilisatoren wie z.B. solche vom Typ der Phosphorigsäureester. Weiterhin können sonstige in der Kunststofftechnologie übliche Zusätze wie z.B. Flammschutzmittel, Antistatika, Weichmacher, Gleitmittel, Treibmittel, Pigmente, Verstärkungsstoffe oder Füllstoffe zugesetzt werden.

Bei der Mitverwendung bekannter Stabilisatoren können synergistische Effekte auftreten, was besonders bei Mitverwendung von anderen Lichtschutzmitteln oder von organischen Phosphiten häufig der Fall ist. Von besonderer Bedeutung ist die Mitverwendung von Antioxydantien bei der Stabilisierung von Polyolefinen.

Die Erfindung betrifft daher auch die durch Zusatz von 0,1 bis 5 Gew.% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden z.B. als Folien, Fasern, Bändchen, Profile oder als Bindemittel für Lacke, Klebemittel oder Kitte.

Die Herstellung und Verwendung der erfindungsgemässen Verbindungen wird in den folgenden Beispielen näher beschrieben. Teile bedeuten darin Gewichtsteile und % Gewichtsprozente. Die Temperaturen sind in Celsius-Graden angegeben.

Beispiele 1-3: Substitution am Piperidin-Stickstoff.

18,2 g (0,05 Mol) 2,2,4,4-Tetramethyl-7-oxa-3,20-diaza-21-oxo-dispiro[5,1,11,2]heneicosan werden in 100 ml Dioxan gelöst. Dazu gibt man 1 g KJ und 7.3 g (0,06 Mol) Allylbromid sowie 9,7 g $K_2CO_3$ und erhitzt auf Rückfluss. Nach 30 Std. Reaktionszeit wird heiss filtriert. Aus dem Filtrat kristallisiert beim Abkühlen die Titelverbindung aus, die aus Ligroin umkristallisiert wird. Smp. 223-26°.

Elementaranalyse: Ber. C = 74,2% H = 10,96% N = 6,92%
Gef.:C = 74,0% H = 11,1% N = 7,0%

Verwendet man anstelle von Allylbromid eine äquimolare Menge Benzylchlorid und verfährt im übrigen wie vorhin beschrieben, so erhält man

3-Benzyl-2,2,4,4-tetramethyl-7-oxa-3,20-diaza-21-oxo-dispiro[5,1,11,2]-heneicosan. Smp. 152-154°.

Elementaranalyse: Ber.: C = 76,60% H = 10,20% N = 6,16%
Gef.: C = 76,83% H = 10,13% N = 6.32%

Löst man die gleiche Menge (0,05 Mol) 2,2,4,4-Tetramethyl-7-oxa-3,20-diaza-21-oxo-dispiro[5,1,11,2]-heneicosan in 50 ml Methanol und lässt die Lösung im Autoklaven bei 120° mit 0,06 Mol Aethylenoxid 30 Std. reagieren, so erhält man 3-Hydroxyäthyl-2,2,4,4-tetramethyl-7-oxa-3,20-diaza-21-oxo-dispiro[5,1,11,2]heneicosan, als weisses Kristallisat. Smp. 260-262° (aus Isopropanol).

| | | | | |
|---|---|---|---|---|
| Elementaranalyse: | Ber.: | C = 70,55% | H = 10,85% | N = 6,85% |
| ($C_{24}H_{44}N_2O_3$) | Gef.: | C = 70,36% | H = 10,68% | N = 6,93% |

<u>Beispiele 4-6:</u> Substitution am Oxazolon-Stickstoff.

18,2 g (0,05 Mol) 2,2,4,4-Tetramethyl-7-oxa-3,20-diaza-21-oxo-dispiro[5,1,11,2]-heneicosan werden in 200 ml abs. Tetrahydrofuran gelöst und mit pulv. KOH (3,5 g 10,06 Mol) versetzt. Dazu gibt man 1,6 g (10 Mol%) Tetrabutylammoniumbromid (PTC) und erwärmt auf 60-70°. Man lässt dazu eine Lösung von 6 g (0,05 Mol) Allylbromid in 20 ml abs. THF langsam zutropfen. Nach dem Zutropfen wird bei gleicher Temperatur 3 Stunden gerührt.Danach wird abgekühlt, filtriert und das Filtrat eingedampft. Der Rückstand wird aus Acetonitril umkristallisiert. Das erhaltene 2,2,4,4-Tetramethyl-7-oxa-3,20-diaza-20-allyl-21-oxo-dispiro[5,1,11,2]-heneicosan ist eine weisse kristalline Substanz, die bei 120-121° schmilzt.

| | | | | |
|---|---|---|---|---|
| Analyse: | Ber.: | C = 74,21% | H = 10,96% | N = 6,92% |
| | Gef.: | C = 74,51% | H = 10,95% | N = 6,94% |

Verwendet man anstelle von Allylbromid eine äquimolare Menge Benzylchlorid und verfährt im übrigen wie vorhin beschrieben, so erhält man

2,2,4,4-Tetramethyl-7-oxa-3,20-diaza-20-benzyl-21-oxo-dispiro[5,1,11,2(heneicosan. Smp. 150-151° (Acetonitril)

| | | | | |
|---|---|---|---|---|
| Elementaranalyse: | Ber.: | C = 76,60% | H 10,20% | N = 6,16% |
| | Gef.: | C = 76,59% | H 10,18% | N = 6,38% |

Beispiel 7: Stabilisierung von Polypropylen gegen Licht.

100 Teile Polypropylenpulver (Moplen, Fibre grade, der Firme Montedison) werden mit 0,2 Teilen β-(3,5-Di-tert-butyl-4-hydroxyphenyl)-propionsäure-octadecylester und 0,25 Teilen eines Stabilisators der folgenden Tabelle 1 im Brabender-Plastographen bei 200°C während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Rohpresslings wird ausgeschnitten und zwischen zwei Hochglanz-Hartaluminiumfolien mit einer handhydraulischen Laborpresse während 6 Minuten bei 260° zu einer 0,1 mm dicken Folie gepresst, die unverzüglich in kaltem Wasser abgeschreckt wird. Aus dieser werden nun Abschnitte ausgestanzt und im Xenotest 1200 belichtet. Zu regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5,85 µm während der Belichtung ist ein Mass für den photoxidativen Abbau des Polymeren (s.L. Balaban et al., J. Polymer Sci, Part C; 22, 1059-1071 (1969))und ist erfahrungsgemäss mit einem Abfall der mechanischen Eigenschaften des Polymeren verbunden. Als Mass der Schutzwirkung gilt die Zeit bis Erreichen einer Carbonylextinktion von ca. 0,3, bei welcher die Vergleichsfolie brüchig ist. Diese Carbonylextinktion haben die erfindungsgemässen Verbindungen nach 5100 h noch nicht erreicht.

Die Schutzwirkung der Stabilisatoren gemäss der Erfindung ist aus folgender Tabelle 1 ersichtlich.

Tabelle 1

Stabilisierung von Polypropylen mit Verbindungen der Formel

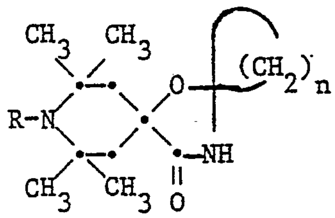

| R = | Belichtungs-zeit | resultierende Carbonylextinktion |
|---|---|---|
| Benzyl | 5100 h | 0,17 |
| Allyl | 5100 h | 0,25 |
| ohne Stabilisator | 800 h | 0,30 |

Patentansprüche

1. Verbindungen der Formel

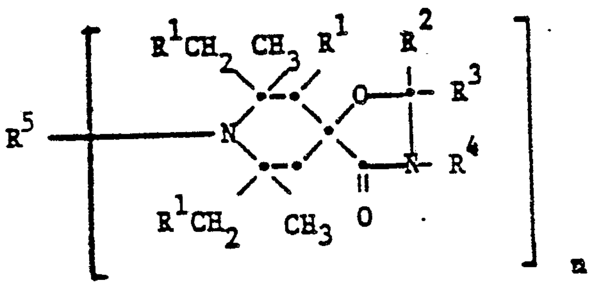

I

und deren Säureadditions-Salze, worin n eine ganze Zahl von 1 bis 4 ist, $R^1$ Wasserstoff oder $CH_3$ ist, $R^2$ und $R^3$ unabhängig voneinander Wasserstoff, $C_1$-$C_{18}$-Alkyl, unsubstituiertes oder durch Chlor oder $C_1$-$C_4$-Alkyl substituiertes $C_6$-$C_{10}$-Aryl oder $C_7$-$C_9$-Aralkyl bedeuten oder $R^2$ und $R^3$ zusammen mit dem C-Atom, an das sie gebunden sind, einen Cycloalkan- oder Alkylcycloalkanrest mit 5-20 C-Atomen oder einen Polyalkylpiperidinrest bilden, $R^4$ Wasserstoff, $C_1$-$C_8$-Alkyl, Allyl, Propargyl, Glycidyl oder unsubstituiertes oder durch $C_1$-$C_4$ substituiertes $C_7$-$C_9$-Aralkyl ist, $R^5$ wenn n=1 ist, Wasserstoff, $C_1$-$C_8$-Alkyl, $C_3$-$C_8$-Alkenyl, $C_3$-$C_5$ Alkinyl, unsubstituiertes oder durch $C_1$-$C_4$-Alkyl substituiertes $C_7$-$C_9$-Aralkyl oder einen Rest $-CH_2-CH(R^6)-OR^7$ darstellt, worin $R^6$ H, $CH_3$, $C_2H_5$ oder Phenyl ist und $R^7$ Wasserstoff oder den Acylrest einer aliphatischen, cycloaliphatischen oder aromatischen Monocarbonsäure mit bis zu 18 C-Atomen bedeutet, und wenn n=2 ist, 1,4-Buten-2-ylen, m- oder p-Xylylen oder einen Rest der Formel $-CH_2CH(R^6)-O-R^8-O-CH(R^6)-CH_2-$ darstellt, worin $R^6$ die oben gegebene Bedeutung hat und $R^8$ den Diacylrest einer aliphatischen, cycloaliphatischen oder aromatischen Dicarbonsäure mit 3-14 C-Atomen bedeutet, und wenn n=3 ist, einen Rest der Formel

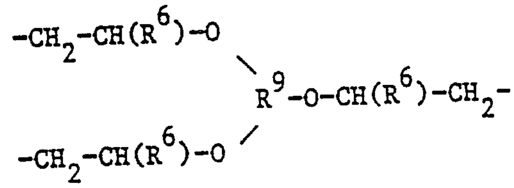

darstellt, worin $R^6$ die oben gegebene Bedeutung hat und $R^9$ den Triacylrest einer aliphatischen oder aromatischen Tricarbonsäure mit 4-18 C-Atomen bedeutet, und wenn n=4 ist, einen Rest der Formel

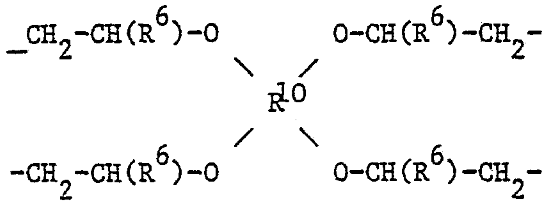

darstellt, worin $R^6$ die oben gegebene Bedeutung hat und $R^{11}$ den Tetracylrest einer aliphatischen oder aromatischen Tetracarbonsäure mit 6-20 C-Atomen bedeutet, wobei im Falle, dass $R^5$ Wasserstoff oder $C_1$-$C_4$-Alkyl ist, $R^4$ nicht Wasserstoff sein kann.

2. Verbindungen gemäss Anspruch 1 der Formel I, worin $R^1$ Wasserstoff ist.

3. Verbindungen gemäss Anspruch 1 der Formel I, worin n 1 oder 2 ist.

4. Verbindungen gemäss Anspruch 1 der Formel I, worin $R^2$ und $R^3$ unabhängig voneinander $C_1$-$C_{12}$-Alkyl oder Phenyl bedeuten oder $R^2$ und $R^3$ zusammen mit dem C-Atom, an das sie gebunden sind, einen $C_6$-$C_{12}$-Cycloalkanring bilden.

5. Verbindungen gemäss Anspruch 1 der Formel I, worin n 1 ist, $R^4$ Wasserstoff ist und $R^5$ Allyl, Benzyl, 2-Hydroxyäthyl oder 2-Hydroxypropyl bedeutet.

6. Verbindungen gemäss Anspruch 1 der Formel I, worin n 1 ist, $R^4$ Allyl oder Benzyl bedeutet und $R^5$ Wasserstoff ist.

7. Verwendung von Verbindungen der Formel I des Anspruches 1 als Lichtstabilisatoren für Kunststoffe.

8. Verwendung gemäss Anspruch 7 in Polyolefinen, Styrolpolymerisaten oder Polyurethanen.

9. Verwendung gemäss Anspruch 7, in einer Menge von 0,01 bis 5 Gew.%, bezogen auf den zu stabilisierenden Kunststoff.

10. Stabilisierter Kunststoff, gekennzeichnet durch einen Gehalt von 0,01 bis 5 Gew.%, bezogen auf den zu stabilisierenden Kunststoff, an mindestens einer Verbindung der Formel I des Anspruches 1.

11. Stabilisierter Kunststoff gemäss Anspruch 10, gekennzeichnet durch den Gehalt sonstiger bekannter und üblicher Zusätze.